# NEUE EUROPÄISCHE PATENTSCHRIFT

(11) **EP 1 674 067 B2**
(45) Veröffentlichungstag und Bekanntmachung des Hinweises auf die Entscheidung über den Einspruch: **08.05.2013**
(45) Hinweis auf die Patenterteilung: 15.04.2009
(21) Anmeldenummer: 05024095.1
(22) Anmeldetag: 04.11.2005
(51) Int. Cl.: A61K 6/00, A61K 6/083

(54) **Hydrolysestabiles selbstätzendes Einkomponentendentaladhäsiv**
Hydrolysis-stable self-etching one-component dental adhesive
Composition d'adhésif dentaire en une partie d'auto-gravure

(30) Priorität: 22.12.2004 DE 102004061924
(43) Veröffentlichungstag der Anmeldung: 28.06.2006
(73) Patentinhaber: Ivoclar Vivadent AG, 9494 Schaan (LI)
(72) Erfinder: Salz, Ulrich, Dr., 88131 Lindau (DE); Mücke, Angela, Dr., 86199 Augsburg (DE); Zimmermann, Jörg, Dr., 8046 Zürich (CH); Moszner, Norbert, Prof. Dr., 9495 Triesen (LI); Zeuner, Frank, Dr., 9488 Schnellenberg (LI); Rheinberger, Volker, Dr., 9490 Vaduz (LI)
(74) Vertreter: UEXKÜLL & STOLBERG

(56) Entgegenhaltungen:
- EP-A- 0 712 622
- EP-A- 1 374 829
- WO-A-03/013444
- WO-A-03/035013
- WO-A-2004/060327
- WO-A-2004/078100
- WO-A-2005/063778

## Beschreibung

Die Erfindung betrifft Zusammensetzungen, die mindestens ein saures (Meth)acrylamidmonomer, das zwei polymerisierbare Gruppen aufweist, und mindestens ein saures, hydrolysestabiles Monomer, das nur eine polymerisierbare Gruppe aufweist, enthalten. Die Zusammensetzungen eignen sich besonders als selbstätzende, selbstkonditionierende Adhäsive für dentale Zwecke.

Selbstätzende, selbstkonditionierende Dentin/Schmelzadhäsive enthalten gewöhnlich ein Monomer mit Säurefunktion, gegebenenfalls ein oder mehrere nicht saure Comonomere und Lösungsmittel. Die US 6,147,137 beschreibt zum Beispiel die Kombination von sauren Monomeren mit hydrophilen Monomeren wie z.B. 2-Hydroxyethylmethacrylat. Als saure Monomere werden polymerisierbare Acrylat- oder Methacrylate mit aromatischen Carbonsäuregruppen eingesetzt.

Aus der US 6,592,372 sind dentale Primer bekannt, die olefinisch ungesättigte Monomere mit endständigen Sulfonsäuregruppen enthalten. Die Primer sollen sich zur Kombination mit herkömmlichen Adhäsiven eignen.

Die EP 0 333 503 A2 offenbart härtbare Zusammensetzungen, die einen Füllstoff enthalten, der mit polymerisierbaren organischen Phosphor- oder Phosphonsäureverbindungen behandelt wurde. Die Zusammensetzungen können zusätzlich acide Monomere enthalten.

Die DE 199 18 974 A1 offenbart Dentalwerkstoffe auf der Basis von polymerisierbaren, ethylenisch ungesättigten Monomeren, die eine Phosphonsäuregruppe aufweisen. Hierbei handelt es sich um Ester der (Meth)acrylsäure.

Aus der EP 0 811 368 A1 sind dentale Primerzusammensetzungen bekannt, die als acide Monomere Verbindungen mit Carboxylgruppen, Phosphorsäuregruppen, Sulfonsäuregruppen oder Phosphonsäuregruppen enthalten. Durch die Verwendung einer Kombination aus Arylborat und Übergangsmetallverbindung als Initiator sollen Primer erhältlich sein, die bei der chemischen Härtung eine hohe Haftung ergeben.

Die DE 101 01 523 A1 offenbart Dentalmaterialien, die neben stark sauren polymerisationsfähigen Monomeren, wie Phosphorsäureestermethacrylaten oder Acrylphosphonsäuren multifunktionelle Amide als Vernetzer enthalten.

Die WO 03/035013 und die WO 03/013444 offenbaren selbstätzende, selbstkonditionierende Dentaladhäsive auf der Basis von Säuregruppen enthaltende (Meth)acrylamiden, wobei Sulfonsäure- und Phosphonsäuregruppen enthaltende Monomere bevorzugt sind.

Die in selbstätzenden, selbstkonditionierenden Dentaladhäsiven enthaltenen aciden Monomeren reagieren stark sauer und begünstigen insbesondere in Anwesenheit von Wasser Hydrolysereaktionen, was die Stabilität der Zusammensetzungen beeinträchtigt. Deshalb werden die aciden Monomere wasserfrei und meist von den anderen Adhäsivbestandteilen getrennt aufbewahrt. Die aciden Monomere werden entweder kurz vor der Anwendung mit den übrigen Komponenten gemischt oder aber getrennt auf die Zahnoberfläche appliziert. Beide Verfahren sind mit der Gefahr behaftet, daß bei der Anwendung Fehler gemacht werden, die zum klinischen Mißerfolg führen. Aus diesem Grund werden Einkomponentenmaterialien bevorzugt. Zwar werden im Stand der Technik bereits selbstätzende, selbstkonditionierende Einkomponentensysteme beschrieben, diese verlieren jedoch nachweislich durch hydrolytischen Abbau ebenfalls ihre Funktion. Besonders problematisch für den Anwender ist dabei, daß er keine Kontrolle über den Fortgang des hydrolytischen Abbaus hat und somit stets eine gewisse Ungewißheit bezüglich des klinischen Erfolgs bei Anwendung derartiger Adhesive besteht.

Der Erfindung liegt demgemäß die Aufgabe zu Grunde, ein selbstätzendes Einkomponentenadhäsiv für die dentale Anwendung zur Verfügung zu stellen, das hydrolysestabil ist und über eine hohe Haftung an Zahnschmelz und Dentin verfügt.

Erfindungsgemäß wird diese Aufgabe durch dentale Zusammensetzungen gelöst, die die folgenden Bestandteile enthalten:
(i) mindestens ein saures (Meth)acrylamidmonomer gemäß der Formel (1), das zwei polymerisierbare Gruppen aufweist,
(ii) mindestens ein saures Monomer gemäß der Formel (2) und/oder der Formel (3), das nur eine polymerisierbare Gruppe aufweist, und
(iii) mindestens einen Polymerisationsinitiator.

Gemäß einer bevorzugten Ausführungsform enthalten die Zusammensetzungen zusätzlich mindestens ein nicht saures (Meth)acrylamidmonomer (iv) und besonders bevorzugt auch ein Lösungsmittel (v). Als Lösungsmittel eignen sich besonders Wasser, mit Wasser mischbare organische Lösungsmittel und Mischungen davon. Bevorzugte mit Wasser mischbare organische Lösungsmittel sind Aceton, Ethanol und Isopropanol.

Die Zusammensetzungen können vorteilhaft weiterhin einen oder mehrere Füllstoffe (vi) zur Einstellung der rheologischen Eigenschaften, zur Verbesserung der mechanischen Eigenschaften sowie als Röntgenkontrastmittel enthalten. Zur Einstellung der rheologischen Eigenschaften und zur Verbesserung der mechanischen Eigenschaften kommen vorzugsweise partikuläre Füllstoffe zum Einsatz, insbesondere kugelförmige, amorphe SiO₂-Partikel oder Mischoxidpartikel aus SiO₂, ZrO₂, Tantaloxid und/oder TiO₂. Vorzugsweise sind diese Partikel mit einem radikalisch polymerisierbaren Silan oberflächenmodifiziert. Als röntgenopaque Füllstoffe werden vorzugsweise Ytterbiumtrifluorid, Bariumsulfat oder Tantaloxid eingesetzt. Die Füllstoffe weisen vorzugsweise eine Teilchengröße im Bereich von 10 - 500 nm auf.

Außerdem können die Zusammensetzungen ein oder mehrere weitere Additive (vii) enthalten. Besonders bevorzugt sind Zusammensetzungen, die als Additiv mindestens einen Inhibitor und/oder Stabilisator enthalten. Vorteilhafte Inhibitoren und Stabilisatoren sind 2,6-Di-tert-butyl-4-cresol, Methylhydrochinon oder Phenothiazin.

Bei den erfindungsgemäß als saure (Meth)acrylamidmonomere (i) eingesetzten Verbindungen handelt es sich um Substanzen mit radikalisch polymerisierbaren Acrylamid- und/oder Methacrylamidgruppen gemäß der Formel (1). Monomere mit zwei oder mehr polymerisierbaren Gruppen fungieren bei der Polymerisation als Vernetzer und werden daher im folgenden auch als vernetzende Monomere bezeichnet. Methacrylamidmonomere sind besonders bevorzugt.

Die Monomere (i) enthalten Monothiophosphorsäure- und ganz besonders bevorzugt Phosphorsäuregruppen. Die Verbindungen können unterschiedliche Säuregruppen oder vorzugsweise identischen Säuregruppen enthalten.

Die (Meth)acrylamide (i) weisen die folgende chemische Struktur auf:

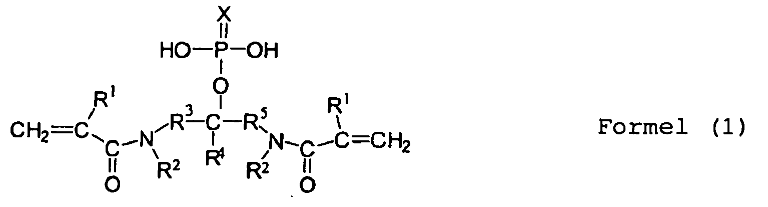

In Formel 1 haben die Variablen die folgenden Bedeutungen:
R¹ = H, CH₃,
R² = H, C₁-C₅-Alkyl,
R³, R⁵ = unabhängig voneinander ein C₁ bis C₁₀ Alkylenrest, vorzugsweise ein C₁ bis C₅ Alkylenrest und insbesondere ein C₁ bis C₃ Alkylenrest, wobei lineare Reste bevorzugt sind,
R⁴ = H oder ein C₁ bis C₁₀ Alkylrest, vorzugsweise ein C₁ bis C₅ Alkylrest.
X = O, S.

Ein besonders bevorzugtes Beispiel für (Meth)acrylamide der Formel (1) ist das 1,3-Dimethacrylamido-propan-2-phosphat (DMAPP):

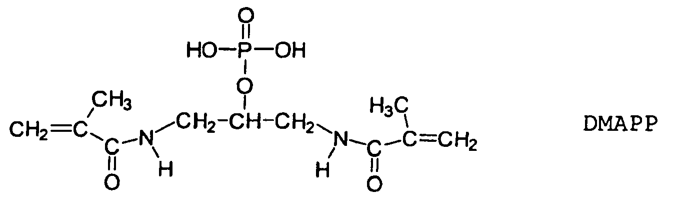

Die erfindungsgemäßen sauren (Meth)acrylamidmonomere (i) der allgemeinen Formel (1) lassen sich ausgehend von kommerziell erhältlichen Diaminoalkoholen in einer 2-stufigen Reaktion herstellen. In der ersten Stufe erfolgt die Umsetzung des primären (R² = H) oder sekundären (R² = C₁-C₅ Alkyl) Diaminoalkohols mit einem funktionellen (Meth)acrylsäurederivat (Chlorid, Ester, Säure oder Anhydrid) unter Anwendung der aus der organischen Chemie bekannten Methoden für die Knüpfung von Amid-Bindungen (vgl. Methoden der Organischen Chemie, Houben-Weyl Band E5 1985, Georg Thieme Verlag Seiten 941 ff). In der zweiten Stufe wird der Bis(meth)acrylamidoalkohol erst mit (Thio)phosphorylhalogenid und nachfolgend in situ mit Wasser zum Dihydrogen(thio)phosphat umgesetzt (vgl. Methoden der Organischen Chemie, HOUBEN-WEYL Band XII/2 1964, Georg Thieme Verlag Stuttgart, Seiten 162 ff).

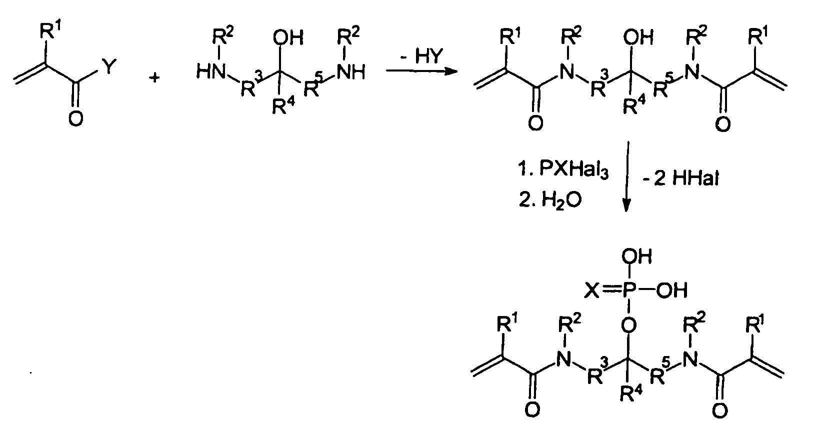

Weiterhin ist es möglich, ausgehend von einem primären (R² = H) oder sekundären (R² = C₁-C₅ Alkyl) Diaminoalkanol zuerst die Phosphorylierung am Sauerstoff durchzuführen und nachfolgend die freie Aminogruppe zu (meth)acrylieren (J. Touet, C. Pierre, E. Brown, Macromol. Chem., Rapid Comm. 8 (1987) 377 sowie J. Touet, C. Pierre, E. Brown, Macromol. Chem. 190 (1989) 313).

Die Monomere (i) zeichnen sich durch eine hohe Hydrolysestabilität aus. Im Zusammenhang mit der vorliegenden Erfindung werden solche Verbindungen als hydrolysestabil bezeichnet, die in Wasser oder in Mischungen von Wasser und wassermischbaren Lösungsmitteln bei einer Konzentration von ca. 20 Gew.-% und einem pH-Wert von ca. 2,0 bei 37 °C für mindestens 6 Wochen stabil sind, d.h. zu weniger als 5 % hydrolysieren.

Bei den erfindungsgemäß als saure Monomere (ii) eingesetzten Verbindungen der Formeln (2) und (3) handelt es sich um Substanzen mit nur einer radikalisch polymerisierbaren (Meth)acrylamidgruppe oder α-substituierten Acrylgruppe. Da die Monomere (ii) nur über eine radikalisch polymerisierbare Gruppe verfügen, haben sie keine vernetzende Wirkung und werden im folgenden daher auch als nicht vernetzende Monomere bezeichnet. Als Monomere (ii) sind hydrolysestabile Verbindungen bevorzugt.

Die Monomere (ii) enthalten vorzugsweise mindestens eine Säuregruppe, besonders bevorzugt 1 bis 2 Säuregruppen. Geeignete Säuregruppen sind Carbonsäure-, Sulfonsäure-, Phosphonsäure- und/oder Phosphorsäuregruppen. Verbindungen, die als acide Gruppe Carbonsäure-, Phosphonsäure- und/oder Phosphorsäuregruppen enthalten, sind besonders bevorzugt. Verbindungen mit mehr als einer Säuregruppen können unterschiedliche Säuregruppen oder vorzugsweise identischen Säuregruppen enthalten.

Die (Meth)acrylamide (ii) weisen die folgende chemischen Strukturen auf:

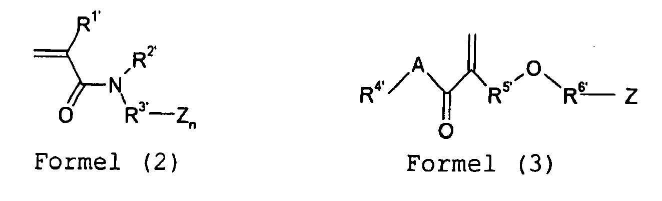

In Formel (2) haben die Variablen die folgenden Bedeutungen:
R^{1'}= H, CH₃,
R^{2'} = H,C₁-C₅ Alkyl oder R^{2'} bildet zusammen mit R^{3'} und dem Stickstoffatom, an das es gebunden ist, einen Heterocyclus mit 5 bis 8, vorzugsweise 6 Ringatomen,
R^{3'}= ein n-fach durch Z substituierter aliphatischer C₁ bis C₁₂ Kohlenwasserstoffrest, vorzugsweise C₂ bis C₆ Kohlenwasserstoffrest, wobei lineare Kohlenwasserstoffreste besonders bevorzugt sind und wobei die Kohlenstoffketten der Kohlenwasserstoffreste durch ein oder mehrere O-Atome unterbrochen sein können oder R^{3'} bildet zusammen mit R^{2'} und dem Stickstoffatom, an das es gebunden ist, einen Heterocyclus mit 5 bis 8, vorzugsweise 6 Ringatomen,
Z =-P(OH)₂(=O), -SO₃H, -COOH, -OP(OH)₂(=S) oder -OP(OH)₂(=O),
n =1 oder 2.

Die Angabe, daß Kohlenwasserstoff-, Alkyl- oder Alkylenreste durch ein oder mehrere Sauerstoffatome unterbrochen sein können, ist so zu verstehen, daß ein oder mehrere Sauerstoffatome in die Kohlenstoffkette der Reste eingeschoben sind. Die Sauerstoffatome sind jeweils an zwei benachbarte Kohlenstoffatome gebunden und können damit nicht endständig sein. Auch können nicht zwei oder mehr Sauerstoffatome aneinander gebunden sein.

Bevorzugte Verbindungen der Formel (2) sind: 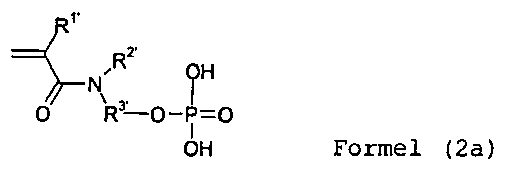 wobei die Variablen wie bei Formel 2 definiert sind. Da n den Wert 1 hat, ist R^{3'} ein Alkylenrest oder bildet zusammen mit R^{2'} und dem Stickstoffatom, an das sie gebunden sind, einen Heterocyclus, wie oben angegeben.

Ein bevorzugtes Beispiel der Formel (2a) ist das 1-Methacrylamido-hexan-6-dihydrogenphosphat (MHP):

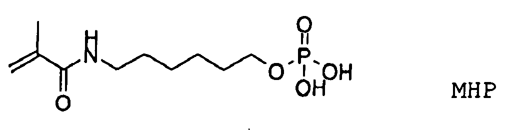

Weitere bevorzugte Verbindungen der Formel (2) sind: 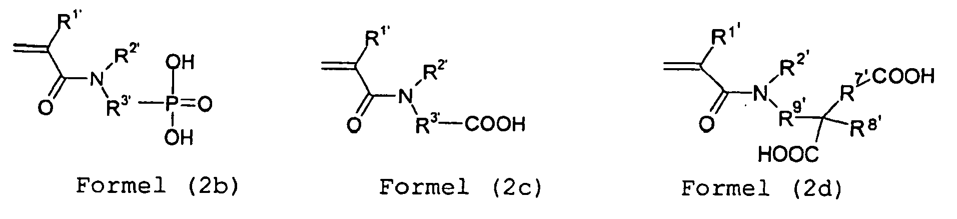 wobei die Variablen R^{1'}, R^{2'} und R^{3'} wie bei Formel (2) und Formel (2a) definiert sind und
R^{7'}=entfällt oder ein C₁ bis C₁₀ Alkylenrest, vorzugsweise ein C₁ bis C₆ Alkylenrest, wobei lineare Alkylenreste besonders bevorzugt sind,
R^{8'}=ein C₁ bis C₁₀ Alkylrest, vorzugsweise ein C₁ bis C₆ Alkylrest, besonders bevorzugt H,
R^{9'}= entfällt oder ein C₁ bis C₁₀ Alkylenrest, vorzugsweise ein C₁ bis C₆ Alkylenrest, wobei lineare Alkylenreste besonders bevorzugt sind.

Ein besonders bevorzugtes Beispiel der Formel (2d) ist die N-Acryloylasparaginsäure (AAA):

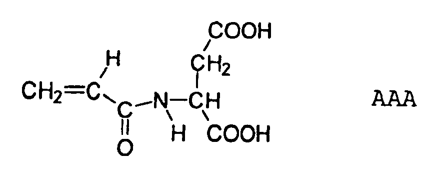

Die sauren Monomere (ii) der allgemeinen Formel (2) lassen sich durch Umsetzung von primären (R^{2'} = H) oder sekundären (R^{2'} = C₁-C₅ Alkyl) Aminen, die eine oder n Säuregruppen Z enthalten, mit funktionellen (Meth)acrylsäurederivaten (Chlorid, Ester, Säure oder Anhydrid) unter Anwendung der aus der organischen Chemie bekannten Methoden für die Knüpfung von Amid-Bindungen (vgl. Methoden der Organischen Chemie, HOUBEN-WEYL Band E5 1985, Georg Thieme Verlag Seiten 941 ff sowie z.B. US 4,157,418) herstellen.

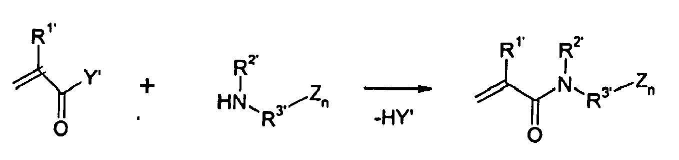

In Formel (3) haben die Variablen die folgenden Bedeutungen:
A =O, S, NR' mit R' = C₁ bis C₆ Alkyl,
R^{4'}= C₁-C₆ Alkyl, ein unsubstituierter oder substituierter Phenylrest,
R^{5'}=ein C₁ bis C₁₂ Alkylenrest, vorzugsweise C₁ bis C₆ Alkylenrest, wobei lineare Alkylenreste besonders bevorzugt sind,
R^{6'}=ein C₁ bis C₁₂ Alkylenrest, vorzugsweise C₂ bis C₆ Alkylenrest, wobei lineare Alkylenreste besonders bevorzugt sind,
Z= -SO₃H, -COOH, -OP(OH)₂(=S), -OP (OH)₂(=O), vorzugsweise -P(OH)₂(=O).

Bei den Substituenten des Phenylrests handelt es sich vorzugsweise um C₁ bis C₆ Alkylgruppen, besonders bevorzugt C₁ bis C₃ Alkylgruppen. Substituierte Phenylreste weisen vorzugsweise 1 bis 3 Substituenten auf.

Ganz besonders bevorzugte Verbindungen der Formel (3) sind: 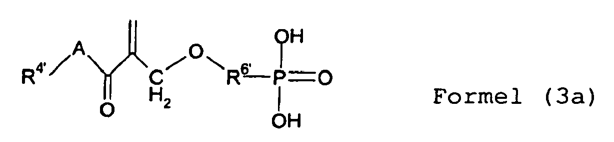 wobei die Variablen die folgenden Bedeutungen haben:
R^{4'}=C₁-C₆ Alkyl, ein unsubstituierter oder substituierter Phenylrest,
R^{6'}=ein C₁ bis C₁₂ Alkylenrest, vorzugsweise C₂ bis C₆ Alkylenrest, wobei lineare Alkylenreste besonders bevorzugt sind.

Besonders bevorzugte Beispiele der Formel (3a) sind Ethyl 2-[4-(dihydroxyphosphoryl)-2-oxabutyl]acrylat (EAEPA) und 2,4,6 Trimethylphenyl 2-[4-(dihydroxyphosphoryl)-2-oxabutyl]acrylat (MAEPA):

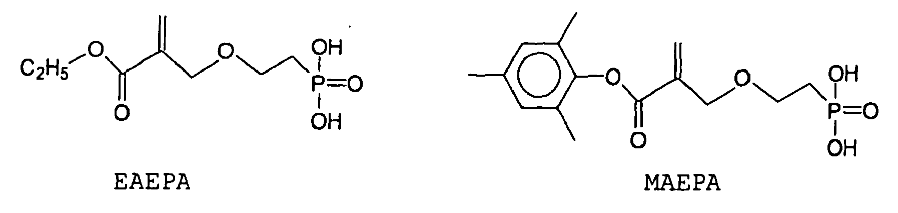

Die erfindungsgemässen sauren Monomere (ii) der allgemeinen Formel (3) lassen sich durch Umsetzung von α-Halogenmethylacrylsäureestern mit geschützten funktionellen Phosphonsäureestern und nachfolgender regioselektiver Hydrolyse, wie in DE 197 46 708 beschrieben, herstellen. Der Austausch von Subtituenten am Carbonsäurerest (z.B. zur Synthese von MAEPA) ist in DE 102 34 326 dokumentiert.

Besonders bevorzugt sind Zusammensetzungen, die als saures Monomer (ii) eine Verbindung der Formel (2) enthalten und ganz besonders bevorzugt solche, die eine Verbindung der Formel (2a) oder (2d) enthalten. Weiter bevorzugt sind Zusammensetzungen, die als saures Monomer (ii) eine Mischung von mindestens zwei verschiedenen Verbindungen gemäß den Formeln (2) und/oder (3) enthalten, insbesondere der Formeln (2a), (2b), (2c) und/oder (2d).

Die erfindungsgemäßen Zusammensetzungen enthalten als nicht acides (Meth)acrylamidmonomer (iv) vorzugsweise ein Monomer mit einer oder mehreren radikalisch polymerisierbaren Gruppen.

Als optionale, nicht saure (Meth)acrylamidmonomere (iv) sind erfindungsgemäß Verbindungen gemäß der allgemeinen Formel (4) besonders bevorzugt: 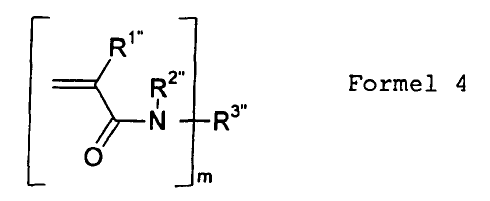 in der
R^{1"}= H, CH₃,
R^{2"}= H, ein C₁ bis C₁₀ Alkylrest, vorzugsweise ein C₁ bis C₆ Alkylrest, oder R^{2"} bildet zusammen mit R^{3"} und dem Stick- stoffatom, an das es gebunden ist, einen Heterocyclus mit 5 bis 8, vorzugsweise 6 Ringatomen,
R^{3"}=ein substituierter aliphatischer C₁ bis C₁₂ Kohlenwasserstoffrest, vorzugsweise C₂ bis C₆ Kohlenwasserstoffrest, wobei lineare Kohlenwasserstoffreste besonders bevorzugt sind und wobei die Kohlenstoffketten der Kohlenwasserstoffreste durch ein oder mehrere O-Atome unterbrochen sein können, ein C₆ bis C₁₂ Arylen- oder Arylrest oder R^{3"} bildet zusammen mit R^{2"} und dem Stickstoffatom, an das es gebunden ist, einen Heterocyclus mit 5 bis 8, vorzugsweise 6 Ringatomen,
m= 1 oder 2.

R^{3"} ist m-fach durch den in Klammern stehenden Rest substituiert. R^{3"} kann allein durch diesen Rest substituiert sein oder zusätzlich weitere Substituenten aufweisen. Als weitere Substituenten sind Hydroxylgruppen bevorzugt, insbesondere 1 bis 4 Hydroxylgruppen.

Wenn m = 1, ist R^{3"} vorzugsweise ein Alkylrest, besonders bevorzugt ein linearer Alkylrest, wobei der Alkylrest durch 1 bis 4, insbesondere 1 bis 2 Hydroxylgruppen substituiert sein kann.

Besonders bevorzugte Verbindungen des Typs m = 1 sind Substanzen, die folgende chemische Struktur aufweisen: 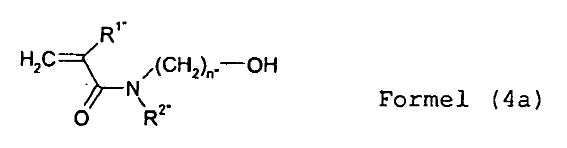 wobei
R^{1"}=H, CH₃
R^{2"}= H, C₁-C₅Alkyl,
n" =1 bis 12, vorzugsweise 2 bis 10.

Bevorzugte Beispiele der Formel (4a) sind N-(6-Hydroxyhexyl)acrylamid (HHA), N-(5-Hydroxypentyl)acrylamid (HPA) und N-(5-Hydroxypentyl)methyacrylamid (HPMA):

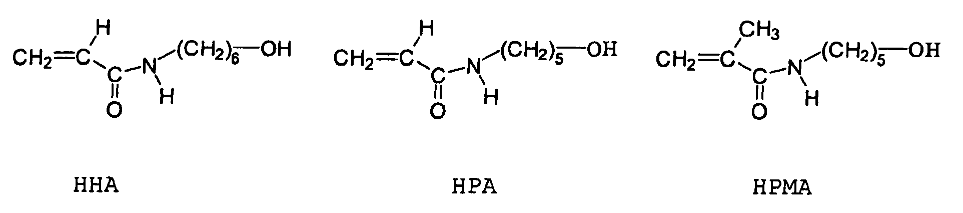

Besonders bevorzugte Verbindungen des Typs m ist gleich 2 sind Substanzen, die folgende chemische Struktur aufweisen: 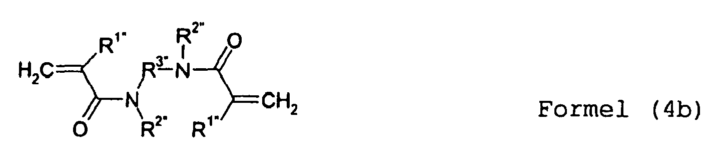 wobei
R^{1"}= H, CH₃
R^{2"}= H, C₁ bis C₁₀ Alkyl, vorzugsweise C₁ bis C₃ Alkyl,
R^{3"}= C₁ bis C₁₀ Alkylen, vorzugsweise C₂ bis C₈, wobei die Alkylenreste durch ein oder mehrere O-Atome unterbrochen sein können.

Bevorzugte Beispiele der Formel (4b) sind N,N'-(Diethyl)-1,3-propylen-bis-acrylamid (DEPBA) und N,N'-(Dimethyl)-1,6-hexylen-bis-acrylamid (DMHBA):

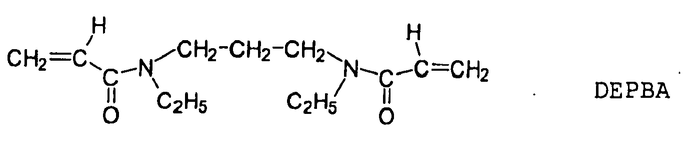

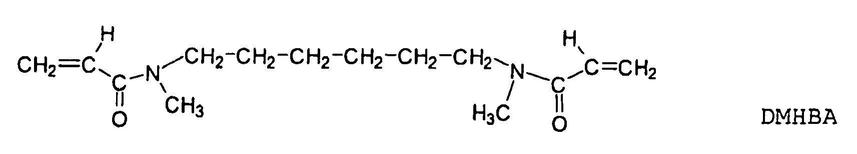

Die erfindungsgemässen (Meth)acrylamidmonomere (iv) der allgemeinen Formel (4) lassen sich durch Umsetzung von primären (R^{2"} = H) oder sekundären (R^{2"} = C₁ bis C₁₀ Alkylrest) Aminen mit einem funktionellen (Meth)acrylsäurederivat (Chlorid, Ester, Säure oder Anhydrid) unter Anwendung der aus der organischen Chemie bekannten Methoden für die Knüpfung von Amid-Bindungen (vgl. Methoden der Organischen Chemie, HOUBEN-WEYL Band E5 1985, Georg Thieme Verlag, Seiten 941 ff) herstellen und sind u.a. für m = 1 in DE 102 28 540 und für m > 1 in DE 101 01 523 beschrieben.

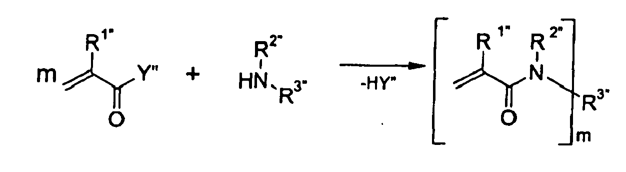

Erfindungsgemäß sind solche Zusammensetzungen besonders bevorzugt, die als (Meth)acrylamid (iv) HPA, HPMA und/oder DEPBA enthalten, sowie Zusammensetzungen, die eine Mischung von Monomeren mit nur einer polymerisierbaren Gruppe und Monomeren mit zwei und mehr polymerisierbaren Gruppen enthalten. Letztere wirken als zusätzliche Vernetzer.

Erfindungsgemäß sind weiterhin solche Zusammensetzungen besonders bevorzugt, die als polymerisierbare Monomere ausschließlich (Meth)acrylamidderivate sowie α-substituierte Acrylate enthalten, insbesondere Monomere der oben angegebenen Typen (i), (ii), (iv). Naturgemäß sind Zusammensetzungen, die die oben definierten bevorzugten Monomere enthalten, ganz besonders bevorzugt.

Zur Initiierung der radikalischen Polymerisation enthalten die Zusammensetzungen vorzugsweise einen Initiator, besonders bevorzugt einen Photoinitiator.

Als Photoinitiatoren werden vorzugsweise Benzophenon, Benzoin sowie deren Derivate oder α-Diketone wie 9, 10-Phenantrenchinon, 1-Phenyl-propan-1,2-dion, Diacetyl oder 4,4-Dichlorbenzil eingesetzt. Besonders bevorzugt werden Campherchinon und 2,2-Methoxy-2-phenyl-acetophenon und insbesondere α-Diketone in Kombination mit Aminen als Reduktionsmittel, wie z.B. 4-(Dimethylamino)-benzoesäureester, N, N-Dimethylaminoethylmethacrylat, N, N-Dimethyl-sym.-xylidin oder Triethanolamin, verwendet.

Die erfindungsgemäßen Zusammensetzungen enthalten die oben angegebenen Komponenten vorzugsweise in den folgenden Mengen, wobei die angegebenen Mengenbereiche getrennt und unabhängig voneinander gewählt werden können:
1 bis 60 Gew.-%, vorzugsweise 10 bis 50 Gew.-% (Meth)acrylamid (i),
1 bis 50 Gew.-%, vorzugsweise 5 bis 40 Gew.-% saures Monomer (ii),
0,01 bis 10 Gew.-%, vorzugsweise 0,1 bis 5 Gew.-% Polymerisationsinitiator (iii),
1 bis 80 Gew.-%, vorzugsweise 10 bis 70 Gew.-% (Meth)acrylamid (iv),
0 bis 80 Gew.-%, vorzugsweise 10 bis 70 Gew.-% Lösungsmittel (v),
0 bis 40 Gew.-%, vorzugsweise 0,5 bis 20 Gew.-% Füllstoff (e) (vi),
0,01 bis 10 Gew.-%, vorzugsweise 0,1 bis 5 Gew.-% Additiv (e) (vii).

Alle Angaben beziehen sich, wenn nicht anders angegeben, auf die Gesamtmasse der Zusammensetzung.

Die Zusammensetzungen weisen vorzugsweise einen pH-Wert im Bereich von 0,5 bis 3,0, besonders bevorzugt 0,5 bis 2,0 auf.

Die erfindungsgemäßen, selbstätzenden, selbstkonditionierenden Einkomponentenadhäsive zeichnen sich durch ihre hohe Hydrolysestabilität sowie ihre gute Haftung an Zahnschmelz und Dentin aus. Sie eignen sich besonders als dentale Adhäsive, insbesondere zur Befestigung von Dentalwerkstoffen an Dentin und Zahnschmelz im Rahmen der restaurativen Zahnmedizin. Besonders eignen sie zur Verwendung mit Dentalwerkstoffen, die durch radikalische Polymerisation ausgehärtet werden, insbesondere Kompositfüllungsmaterialien, Kompositbefestigungsmaterialien, Fissurenversiegler, Brackettzemente, Hybridglasionomere (methacrylatverstärkt) sowie radikalisch polymerisierende Versieglungs- und Beschichtungsmaterialien.

Im folgenden wird die Erfindung anhand von Ausführungsbeispielen weiter erläutert.

### Ausführungsbeispiele

### Beispiele 1 bis 6: Herstellung von Dentaladhäsiven

Durch Mischen der Ausgangskomponenten wurden Adhäsive mit den in Tabelle 1 angegebenen Zusammensetzungen hergestellt. Hierzu wurde zunächst der Füllstoff in dem Lösungsmittel dispergiert. Die Initiatoren/Stabilisatoren wurden in einer Mischung aus flüssigem Monomer und Lösungsmittel gelöst. Durch anschließendes Zusammengeben aller Bestandteile wurde unter Rühren eine homogene Adhäsivmischung erhalten, wobei sich feste Monomere in der Mischung lösten.

Anschließend wurde die Haftung der Adhäsive an Zahnschmelz und Dentin ermittelt. Dazu wurden Rinderzähne so in Kunststoffzylinder eingebettet, daß sich das Dentin bzw. der Zahnschmelz und der Einbettungskunststoff in einer Ebene befanden. Mit einem Microbrush wurde eine Schicht des jeweiligen Adhäsivs 30 s in die zu behandelnde Oberfläche einmassiert, mit einem Luftbläser verblasen und 20 s mit einer herkömmlichen Dentalpolymerisationslampe belichtet. Auf die Adhäsivschicht wurde mit Hilfe einer Delrin Form ein Pfropf aus 2 Schichten eines dentalen Komposits (Tetric Ceram, Ivoclar Vivadent AG) aufgebracht, wobei jede Kompositschicht jeweils für 40 s belichtet wurde. Anschließend wurden die Prüfkörper für 24 h bei 37 °C in Wasser gelagert und dann die Scherhaftfestigkeit gemäß der ISO-Richtlinie "ISO 1994-ISO TR 11405: Dental Materials Guidance on Testing of Adhesion to Tooth Structure" bestimmt. Die gefundenen Ergebnisse sind ebenfalls in Tabelle 1 angegeben.

### Beispiele 7 bis 9: Herstellung von Dentaladhäsiven (Vergleich)

Analog zu den Beispielen 1 bis 6 wurden dentale Adhäsive hergestellt, die entweder nur ein (Meth)acrylatmonomer (i) (Beispiel 8) oder ein Säuremonomer (ii) enthielten (Beispiele 7 und 9). Die Zusammensetzungen der Adhäsive sind in Tabelle 2 angegeben.

Anschließend wurde wie bei den Beispielen 1 bis 6 beschrieben die Haftung an Zahnschmelz und Dentin ermittelt. Die gefundenen Ergebnisse sind ebenfalls in Tabelle 2 gezeigt.

Tabelle 1 läßt erkennen, daß die erfindungsgemäßen Zusammensetzungen eine vorteilhafte Kombination von Eigenschaften haben. Neben einer guten Hydrolysestabilität haben sie eine hohe Haftwirkung auf Zahnschmelz und Dentin. Davon abweichende Zusammensetzungen (Tabelle 2) zeigen zwar ebenfalls eine gute Hydrolysestabilität, haben aber eine deutlich geringere Haftwirkung.

**Tabelle 1**

| **Bestandteil** | | **Bsp. 1** **[Gew.%]** | **Bsp. 2** **[Gew.%]** | **Bsp. 3** **[Gew.%]** | **Bsp. 4** **[Gew.%]** | **Bsp. 5** **[Gew.%]** | **Bsp. 6** **[Gew.%]** |
|---|---|---|---|---|---|---|---|
| vernetzendes acides Monomer (i) | DMAPP | 15,0 | 14,6 | 9,0 | 12,0 | 14,0 | 12,0 |
| nicht vernetzendes acides Monomer (ii) | MHP | 10,0 | - | - | - | - | - |
| | AAA | - | 9,7 | 10,0 | 8,0 | - | 8,0 |
| | MAEPA | - | - | - | - | 9,0 | - |
| vernetzendes nicht acides Monomer (iv) | DEPBA | 49,07 | 47,77 | 36,07 | 39,0 | - | 39,0 |
| | DMHBA | - | - | - | - | 46,0 | - |
| nicht vernet zendes nicht acides Monomer (iv) | HHA | - | 2,0 | 15,0 | - | - | - |
| | HPMA | - | - | - | 15,0 | 5,0 | - |
| | HPA | - | - | - | - | - | 15,0 |
| Lösungsmittel | Wasser | 24,0 | 24,0 | 21,0 | 24,0 | 24,0 | 24,0 |
| | Aceton | - | - | 7,0 | - | - | - |
| Füllstoff | A200 | 1,0 | 1,0 | 1,0 | 1,0 | 1,0 | 1,0 |
| Initiator / Stabilisator | BHT | 0,03 | 0,03 | 0,03 | 0,03 | 0,03 | 0,03 |
| | EMBO | 0,5 | 0,5 | 0,5 | 0,5 | 0,5 | 0,5 |
| | CC | 0,4 | 0,4 | 0,4 | 0,4 | 0,4 | 0,4 |

| **Haftwert** | | **[MPa]** | **[MPa]** | **[MPa]** | **[MPa]** | **[MPa]** | **[MPa]** |
|---|---|---|---|---|---|---|---|
| Schmelz | | 17,4 | 21,7 | 21,0 | 16,9 | 13,8 | 17,9 |
| Dentin | | 23,2 | 23,8 | 21,3 | 21,0 | 19,1 | 19,1 |

| | | | | | | | |
|---|---|---|---|---|---|---|---|
| Abkürzungen: A200 = Aerosil A200 Degussa; BHT = 2,6-Di-tert-butyl-4-cresol; CC = Campherchinon; EMBO = Ethyl-4-dimethyl-aminobenzoat | | | | | | | |

**Tabelle 2**

| **(Vergleichsbeispiele)** | | | | |
|---|---|---|---|---|
| **Bestandteil** | | **Bsp. 7** **[Gew.%]** | **Bsp. 8** **[Gew.%]** | **Bsp. 9** **[Gew.%]** |
| vernetzendes acides Monomer (i) | DMAPP | - | 37,6 | - |
| nicht vernetzendes acides Monomer (ii) | AHP | 20,9 | - | - |
| | AAA | - | - | 15,07 |
| vernetzendes nicht acides Monomer (iv) | DEPBA | 32,6 | - | 19,0 |
| nicht vernetzendes nicht acides Monomer (iv) | HHA | 12,4 | 32,4 | 25,0 |
| Lösungsmittel | Wasser | 18,2 | 28,07 | 30,0 |
| | Aceton | - | - | 9,0 |
| | Ethanol | 13,97 | - | - |
| Füllstoff | A200 | 1,0 | 1,0 | 1,0 |
| Initiator / Stabilisator | BHT | 0,03 | 0,03 | 0,03 |
| | EMBO | 0,5 | 0,5 | 0,5 |
| | CC | 0,4 | 0,4 | 0,4 |

| **Haftwert** | | **[MPa]** | **[MPa]** | **[MPa]** |
|---|---|---|---|---|
| Schmelz | | 9,4 | 7,7 | 7,0 |
| Dentin | | 12,3 | 13,8 | 11,3 |

| | | | | |
|---|---|---|---|---|
| Abkürzungen: A200 = Aerosil A200 Degussa; BHT = 2,6-Di-tert-butyl-4-cresol; CC = Campherchinon; EMBO = Ethyl-4-dimethyl-aminobenzoat | | | | |

## Patentansprüche

1. Dentale Zusammensetzung enthaltend
(i) mindestens ein saures (Meth)acrylamidmonomer, das zwei polymerisierbare Gruppen aufweist, gemäß der folgenden allgemeinen Formel (1): 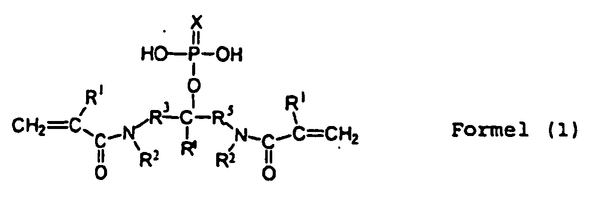 in der
R¹ = H, CH₃,
R² = H, C₁-C₅Alkyl,
R³, R⁵ = unabhängig voneinander ein C₁ bis C₁₀ Alkylenrest,
R⁴ = H oder ein C₁ bis C₁₀ Alkylrest ,
X = O, S;
(ii) mindestens ein saures Monomer, das nur eine polymerisierbare Gruppe aufweist, gemäß der folgenden Formel (2): 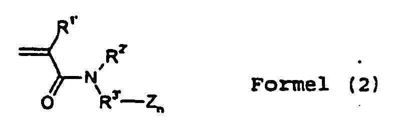 in der
R^{1'} = H, CH₃,
R^{2'} = H, C₁-C₅Alkyl oder R^{2'} bildet zusammen mit R^{3'} und dem Stickstoffatom, an das es gebunden ist, einen Heterocyclus mit 5 bis 8 Ringatomen,
R^{3'} = ein n-fach durch Z substituierter aliphatischer C₁ bis C₁₂ Kohlenwasserstoffrest, wobei die Kohlenstoffketten der Kohlenwasserstoffreste durch ein oder mehrere O-Atome unterbrochen sein können oder R^{3'} bildet zusammen mit R^{2'} und dem Stickstoffatom, an das es gebunden ist, einen Heterocyclus mit 5 bis 8 Ringatomen,
Z = -P(OH)₂(=O), -SO₃H, -COOH, -OP (OH) ₂ (=S) oder -OP(OH)₂(=O) ;
n = 1 oder 2
und/oder gemäß der Formel (3): 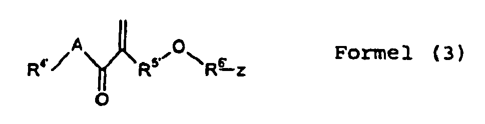 in der
A = O, S, NR mit R' = C₁ bis C₆ Alkyl,
R⁴ = C₁-C₆ Alkyl, ein unsubstituierter oder substituierter Phenylrest,
R^{5'} = ein C₁ bis C₁₂ Alkylenrest,
R^{6'} = ein C₁ bis C₁₂ Alkylenrest,
Z = -SO₃H, -COOH, -OP(OH)₂(=S), -OP(OH)₂(=O) oder -P(OH)₂(=O); und
(iii) mindestens einen Polymerisationsinitiator.

2. Zusammensetzung nach Anspruch 1, die zusätzlich mindestens ein nicht saures (Meth)acrylamidmonomer (iv) enthält.

3. Zusammensetzung nach Anspruch 1 oder 2, die zusätzlich Lösungsmittel (v) enthält.

4. Zusammensetzung nach Anspruch 3, die als Lösungsmittel Wasser und/oder ein mit Wasser mischbares organisches Lösungsmittel enthält.

5. Zusammensetzung nach einem der vorhergehenden Ansprüche, die zusätzlich mindestens einen Füllstoff (vi) enthält.

6. Zusammensetzung nach einem der vorhergehenden Ansprüche, die zusätzlich als Additiv (vii) mindestens einen Inhibitor und/oder Stabilisator enthält.

7. Zusammensetzung nach einem der Ansprüche 1 bis 6, die als (Meth)acrylamid (ii) eine Mischung aus mindestens zwei verschiedenen Monomeren der Formel (2) enthält.

8. Zusammensetzung nach einem der Ansprüche 2 bis 7, die als nicht acides (Meth)acrylamidmonomer (iv) ein Monomer mit einer oder mehreren radikalisch polymerisierbaren Gruppen enthält.

9. Zusammensetzung nach Anspruch 8, in der das (Meth)-acrylamid (iv) eine Verbindung gemäß der allgemeinen Forme 1 (4) ist: in der 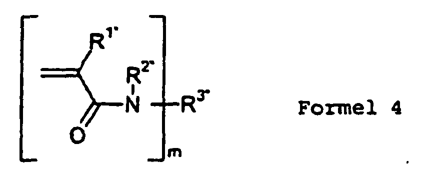
R^{1"} = H, CH₃,
R^{2"} =H, ein C₁ bis C₁₀ Alkylrest oder R^{2"} bildet zusammen mit R^{3"} und dem Stickstoffatom, an das es gebunden ist, einen Heterocyclus mit 5 bis 8 Ringatomen,
R^{3"} = ein substituierter aliphatischer C₁ bis C₁₂ Kohlenwasserstoffrest, wobei die Kohlenstoffketten der Kohlenwasserstoffreste durch ein oder mehrere O-Atome unterbrochen sein können, ein C₆ bis C₁₂ Arylen- oder Arylrest oder R^{3"} bildet zusammen mit R^{2"} und dem Stickstoffatom, an das es gebunden ist, einen Heterocyclus mit 5 bis 8 Ringatomen,
m = 1 oder 2.

10. Zusammensetzung nach Anspruch 8 oder 9, die als (Meth)acrylamid (iv) eine Mischung von Monomeren mit nur einer polymerisierbaren Gruppe und Monomeren mit zwei und mehr polymerisierbaren Gruppen enthält.

11. Zusammensetzung nach einem der vorhergehenden Ansprüche, die
1 bis 60 Gew.-% (Meth)acrylamid (i),
1 bis 50 Gew.-% saures Monomer (ii),
0,01 bis 10 Gew.-% Polymerisationsinitiator (iii),
1 bis 80 Gew.-% (Meth)acrylamid (iv),
0 bis 80 Gew.-% Lösungsmittel (v)
0 bis 40 Gew.-% Füllstoff(e) (vi)
enthält.

12. System aus einer Zusammensetzung gemäß einem der Ansprüche 1 bis 18 und einem durch radikalische Polymerisation härtbarem Dentalwerkstoff.

13. System nach Anspruch 12 bei dem der Dentalwerkstoff ein Kompositfüllungsmaterial, Kompositbefestigungsmaterial, Fissurenversiegler, Brackettzement, methacrylatverstärktes Hybridglasionomer, ein Versieglungsmaterial oder Beschichtungsmaterial ist.

14. Verwendung einer Zusammensetzung gemäß einem der Ansprüche 1 bis 11 zur Befestigung eines radikalisch polymerisierbaren Dentalwerkstoffs an Zahnschmelz und/oder Dentin.

## Claims

1. Dental composition comprising
(i) at least one acidic (meth)acrylamide monomer, which has two polymerisable groups, according to the general Formula (1) below: 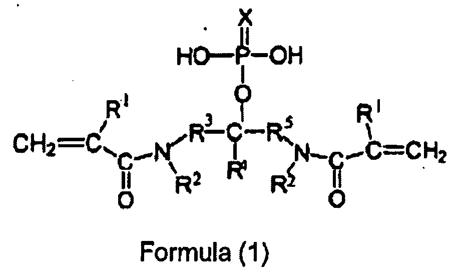 in which
R¹ = H, CH₃,
R² = H, C₁-C₅ alkyl,
R³ R⁵ = independently of each other a C₁ to C₁₀ alkylene group,
R⁴ = H or a C₁ to C₁₀ alkyl group,
X = O, S;
(ii) at least one acidic monomer, which has only one polymerisable group, according to the Formula (2) below: 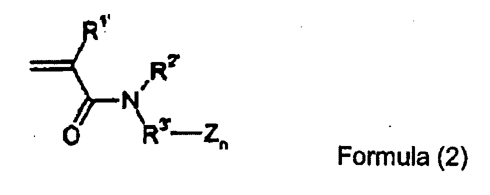 in which
R¹ = H, CH₃,
R^{2'} = H, C₁-C₅ alkyl or R^{2'} forms together with R^{3'} and the nitrogen atom to which it is bonded, a heterocycle with 5 to 8 ring atoms,
R^{3'} = a C₁ to C₁₂ aliphatic hydrocarbon group substituted n fold by Z, wherein the hydrocarbon chains of the hydrocarbon groups can be interrupted by one or more O atoms, or R^{3'} forms, together with R^{2'} and the nitrogen atom to which it is bonded, a heterocycle with 5 to 8 ring atoms,
Z = -P(OH)₂(=O), -SO₃H, -COOH, -OP(OH)₂(=S) or -OP(OH)₂(=O),
n = 1 or 2.
and/or according to Formula (3): 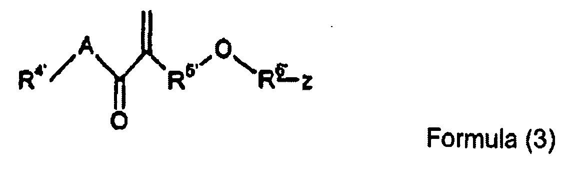 in which
A = O, S, NR' with R' = C₁ to C₆ alkyl,
R^{4'} = C₁-C₆ alkyl, an unsubstituted or substituted phenyl group,
R^{5'} = a C₁ to C₁₂ alkylene group,
R^{6'} = a C₁ to C₁₂ alkylene group,
Z = -SO₃H, -COOH, -OP(OH)₂(=S), -OP(OH)₂(=O), or -P(OH)₂(=O);
and
(iii) at least one polymerisation initiator.

2. Composition according to claim 1, additionally comprising at least one non-acidic (meth)acrylamide monomer (iv).

3. Composition according to claim 1 or 2, additionally comprising solvent (v).

4. Composition according to claim 3, comprising water and/or a water-miscible organic solvent as the solvent.

5. Composition according to one of the preceding claims, additionally comprising at least one filler (vi).

6. Composition according to one of the preceding claims, additionally comprising at least one inhibitor and/or stabiliser as the additive (vii).

7. Composition according to one of claims 1 to 6, comprising a mixture of at least two different monomers of Formula (2) as the (meth)acrylamide (ii).

8. Composition according one of claims 2 to 7, comprising a monomer with one or more radically polymerisable groups as the non-acidic (meth)acrylamide monomer (iv).

9. Composition according to claim 8, in which the (meth)acrylamide (iv) is a compound of the general Formula (4): 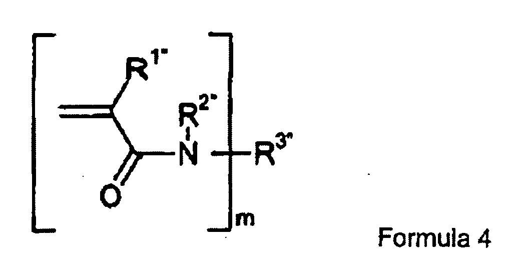 in which
R^{1"} = H, CH₃,
R^{2"} = H, a C₁ to C₁₀ alkyl group, or R^{2"} forms together with R^{3"} and the nitrogen atom to which it is bonded, a heterocycle with 5 to 8 ring atoms,
R^{3"} = a substituted aliphatic C₁ to C₁₂ hydrocarbon group,
wherein the carbon chains of the hydrocarbon groups can be interrupted by one or more O atoms, a C₆ to C₁₂ arylene or aryl group or R^{3"} forms together with R^{2"} and the nitrogen atom to which it is bonded a heterocycle with 5 to 8 ring atoms,
m = 1 or 2.

10. Composition according to claim 8 or 9, comprising a mixture of monomers with only one polymerisable group and monomers with two and more polymerisable groups as the (meth)acrylamide (iv).

11. Composition according to one of the previous claims, comprising
1 to 60 wt.% (meth)acrylamide (i),
1 to 50 wt.% acidic monomer (ii),
0.01 to 10 wt.% polymerisation initiator (iii),
1 to 80 wt.% (meth)acrylamide (iv),
0 to 80 wt.% solvent (v)
0 to 40 wt.% filler(s) (vi).

12. System comprising a composition according to one of claims 1 to 18 and a dental material that is curable by radical polymerisation.

13. System according to claim 12 in which the dental material is a composite filling material, composite fixing material, fissure sealant, bracket cement, methacrylate-reinforced hybrid glass ionomer, a sealing material or coating material.

14. Use of a composition according to one of claims 1 to 11 for fixing a radically polymerisable dental material to tooth enamel and/or dentin.

## Revendications

1. Composition dentaire comprenant
(i) au moins un monomère (méth)acrylamide acide, portant deux groupes polymérisables et répondant à la formule générale (1) suivante : 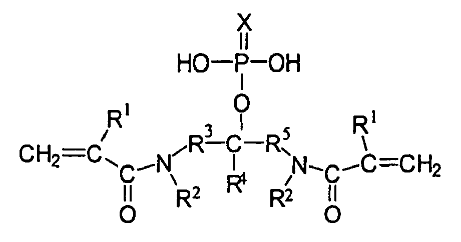 dans laquelle
- R¹ représente un atome d'hydrogène ou un groupe méthyle,
- R² représente un atome d'hydrogène ou un groupe alkyle en C₁ à C₅,
- R³ et R⁵ représentent chacun, indépendamment l'un de l'autre, un groupe alcanediyle en C₁ à C₁₀,
- R⁴ représente un atome d'hydrogène ou un groupe alkyle en C₁ à C₁₀,
- X représente un atome d'oxygène ou de soufre ;
(ii) au moins un monomère acide portant un seul groupe polymérisable et répondant à la formule (2) suivante : 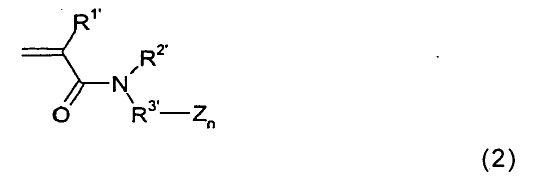 dans laquelle
- R^{1'} représente un atome d'hydrogène ou un groupe méthyle,
- R^{2'} représente un atome d'hydrogène ou un groupe alkyle en C₁ à C₅, ou bien l'entité symbolisée par R^{2'} forme, conjointement avec l'entité symbolisée par R^{3'} et l'atome d'azote auquel elle est liée, un hétérocycle comportant 5 à 8 atomes,
- R^{3'} représente un groupe hydrocarboné aliphatique en C₁ à C₁₂ portant n substituant(s) symbolisé(s) par Z, les chaînes carbonées des groupes hydrocarbonés pouvant être interrompues par un ou plusieurs atome(s) d'oxygène, ou bien l'entité symbolisée par R^{3'} forme, conjointement avec l'entité symbolisée par R^{2'} et l'atome d'azote auquel elle est liée, un hétérocycle comportant 5 à 8 atomes,
- Z représente un groupe de formule -P(=O)(OH)₂, -SO₃H, -COOH, -OP(=S)(OH)₂ ou -OP(=O)(OH)₂,
- l'indice n vaut 1 ou 2,
et/ou répondant à la formule (3) : 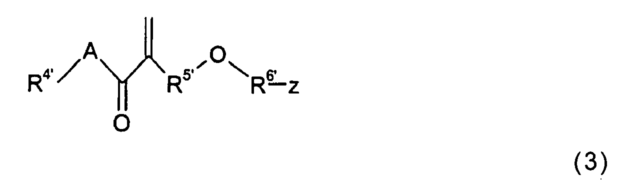
dans laquelle
- A représente un atome d'oxygène ou de soufre, ou une entité de formule NR' où R' désigne un groupe alkyle en C₁ à C₆,
- R^{4'} représente un groupe alkyle en C₁ à C₆ ou un groupe phényle avec ou sans substituant(s),
- R^{5'} représente un groupe alcanediyle en C₁ à C₁₂,
- R^{6'} représente un groupe alcanediyle en C₁ à C₁₂,
- Z représente un groupe de formule -SO₃H, -COOH, -OP(=S)(OH)₂, -OP(=O)(OH)₂ ou -P(=O)(OH)₂ ;
(iii) et au moins un amorceur de polymérisation.

2. Composition conforme à la revendication 1, qui contient en outre (iv) au moins un monomère (méth)acrylamide non-acide.

3. Composition conforme à la revendication 1 ou 2, qui contient en outre (v) un solvant.

4. Composition conforme à la revendication 3, dans laquelle le solvant est de l'eau et/ou un solvant organique miscible à l'eau.

5. Composition conforme à l'une des revendications précédentes, qui contient en outre (vi) au moins une charge.

6. Composition conforme à l'une des revendications précédentes, qui contient en outre (vii) un additif constitué par au moins un inhibiteur et/ou un agent stabilisant.

7. Composition conforme à l'une des revendications 1 à 6, dans laquelle le (méth)acrylamide (ii) est un mélange d'au moins deux monomères de formule (2) différents.

8. Composition conforme à l'une des revendications 2 à 7, dans laquelle le monomère (méth)acrylamide (iv) non-acide est un monomère portant un ou plusieurs groupe(s) polymérisable(s) par voie radicalaire.

9. Composition conforme à la revendication 8, dans laquelle le (méth)acrylamide (iv) est un composé répondant à la formule générale (4) : 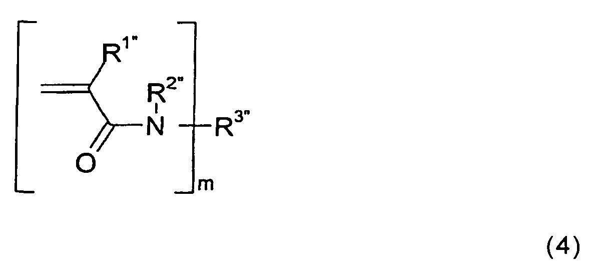 dans laquelle
- R^{1"} représente un atome d'hydrogène ou un groupe méthyle,
- R^{2"} représente un atome d'hydrogène ou un groupe alkyle en C₁ à C₁₀, ou bien l'entité symbolisée par R^{2"} forme, conjointement avec l'entité symbolisée par R^{3"} et l'atome d'azote auquel elle est liée, un hétérocycle comportant 5 à 8 atomes,
- R^{3"} représente un groupe hydrocarboné aliphatique en C₁ à C₁₂ portant un ou plusieurs substituant(s), les chaînes carbonées des groupes hydrocarbonés pouvant être interrompues par un ou plusieurs atome(s) d'oxygène, un groupe arylène ou aryle en C₆ à C₁₂, ou bien l'entité symbolisée par R^{3"} forme, conjointement avec l'entité symbolisée par R^{2"} et l'atome d'azote auquel elle est liée, un hétérocycle comportant 5 à 8 atomes,
- l'indice m vaut 1 ou 2.

10. Composition conforme à l'une des revendications 8 ou 9, dans laquelle le (méth)acrylamide (iv) est un mélange de monomères portant un seul groupe polymérisable et de monomères portant deux et plus de deux groupes polymérisables.

11. Composition conforme à l'une des revendications précédentes, qui contient :
- 1 à 60 % en poids de (méth)acrylamide (i),
- 1 à 50 % en poids de monomère acide (ii),
- 0,01 à 10 % en poids d'amorceur de polymérisation (iii),
- 1 à 80 % en poids de (méth)acrylamide (iv),
- 0 à 80 % en poids de solvant (v),
- 0 à 40 % en poids de charge(s) (vi).

12. Système constitué d'une composition conforme à l'une des revendications 1 à 18 et d'une substance dentaire durcissable par polymérisation radicalaire.

13. Système conforme à la revendication 12, pour lequel la substance dentaire est un matériau composite d'obturation, un matériau composite de fixation, un agent de scellement de fissures, un ciment pour brackets, un ionomère de verre hybride renforcé par méthacrylate, un matériau de scellement ou un matériau de revêtement.

14. Emploi d'une composition conforme à l'une des revendications 1 à 11, pour fixer une substance dentaire polymérisable par voie radicalaire, sur l'émail et/ou la dentine.
